# EUROPEAN PATENT APPLICATION

(11) **EP 4 039 169 A2**
(43) Date of publication of application: **10.08.2022**
(21) Application number: 21218451.9
(22) Date of filing: 31.12.2021
(51) Int. Cl.: A61B 1/31, A61B 5/00, A61B 1/00, A61B 1/07

(54) **SYSTEM FOR SCREENING, DIAGNOSIS, OR MONITORING A COLORECTAL TISSUE AND METHOD THEREOF**

(30) Priority: 14.01.2021 PT 2021117009
(71) Applicant: Universidade do Minho, 4704-553 Braga (PT)
(72) Inventor: GOMES CORREIA, José Higino, 4710-494 Braga (PT); DE JESUS CORREIA MACIEL, Marino, 4905-072 Barcelos (PT)
(74) Representative: Patentree

(57) **Abstract**

The present invention refers to an analysis system of colorectal tissues based on the integration of the optical coherence tomography technique (OCT) in conventional colonoscopy and method thereof. Said system allows the improvement of analysis, diagnosis and monitoring of colorectal tissues. More specifically, it allows the performance of an optical biopsy, without the need to remove the suspicious tissues in colonoscopy, through an in-depth analysis. Furthermore, a polarizing beamsplitter element may be coupled to said system, which allows to increase the contrast of the OCT images in order to better distinguish the type of tissue and its morphology. Said system uses OCT in the spectral domain paired in the colonoscope instrument channel through optical fibre.

## Description

### TECHNICAL FIELD

The present disclosure refers to an analysis system of colorectal tissues which allows the screening, diagnosis, monitoring and minimally invasive intervention in colorectal tissues, through the integration of the optical coherence tomography technique into a conventional device, providing the performance of an optical biopsy.

### BACKGROUND

Currently, the analysis and diagnosis of colorectal tract tissues is performed through a routine colonoscopy exam. The identification of suspicious tissues implies their removal for subsequent biopsy, through a histopathological analysis. This identification is dependent on the practitioner's opinion and subjective analysis, which may lead to false positives and false negatives.

The optical coherence tomography imaging technique (OCT) is one of the greatest inventions of the last three decades, with a major impact on the medical area. Despite being widely used in the ophthalmologic field, it has been directed towards other areas, through the development of different miniaturized versions.

Document US 6,384,915 B1 discloses a device to be incorporated into catheters, endoscopes or other medical devices to measure the location, thickness and structure of arterial walls or other regions. Its main purpose is to be an auxiliary instrument in minimally invasive medical procedures. This apparatus is based on the OCT reflectometry technique and the information collected via this device is used not only to guide the device through the body, but also to evaluate the tissue through which the device is being passed. It is based on the use of multiple optical fibres, arranged in a circular geometry. Light from the distal end of each fibre is directed onto the interior thereof via small-diameter optics. The light reflected or scattered from the cavity walls is then collected by the multiple fibres which are in the sample arm in a typical OCT interferometer. The interferometric result collected sequentially in the different fibres can be used to locate small abnormalities in the arterial walls or cavities (such as aneurysms or arteriovenous malformations) which are not visible in the available techniques. The device is also able to provide information about branching of arteries, necessary for guiding of medical devices through the arterial system. Since only the periphery of this catheter is used for detecting the OCT signal, this means there is room in the central region of the catheter for using other diagnostic instruments and surgical instruments. This device can be incorporated into standard medical catheters, endoscopes or other medical equipment. Similarly, it may also be implemented in non-medical inspection instruments.

Meanwhile, the device disclosed in US 6,384,915 B1 is silent about using a conventional colonoscope for direct integration of the OCT technique in the instrument channel, in order to allow a real-time and non-invasive analysis and diagnosis. This is a generic device which may be incorporated in different catheters, not specifically used in a particular application, such as colonoscopy. Furthermore, it may be incorporated in non-medical equipment as well. Therefore, a person skilled in the art realizes that the lack of equipment specificity may influence the type of analysis performed according to the application intended for the catheter.

Document US 8,983,580 B2 provides a method of forming an image of tissues, initially involving an invasive procedure for exposing the tissue being analysed. After being exposed, low coherence interferometry and OCT data are acquired for subsequent analysis. The method ends with the completion of the invasive procedure, after collection of data volume for analysis, the OCT data collected is converted into at least one image. The method further includes classifying the exposed tissue by using an OCT database and classification algorithms. The document further discloses a system for real-time tissue imaging. The system includes a core imaging unit which generates the optical low coherence interferometry signal and a device which is in communication therewith. The OCT data is generated in this core imaging unit, the system further including a software unit that receives the data and carries out the respective analysis. This invasive method includes removing the tissue, if it is classified as tumoural during data analysis.

Although said document presents a real-time tissue imaging system, it becomes disadvantageous as it requires an invasive procedure for exposing the tissue. Said document doesn't mention, nor does it suggest, an optical biopsy system in addition to a complete OCT analysis, including a polarisation dependent OCT analysis.

Documents US 8,996,099 B2 and US 2015/0265152 A1 present a method based on the use of the OCT technique for identifying pathological structures in the vulnerable plaque. Specifically, it refers to a catheter for image acquisition in patients, which integrates a rotation system at the end of the probe. The probe includes a conduit through which electromagnetic energy is transmitted. This includes a first portion which extends to the conduit, and a second portion which rotates relative to the conduit to redirect the light. The rotating part is driven by a biocompatible gas or liquid, pumped externally. The optical fibre of this system does not rotate, only the prism of the rotating part, which will guide incident light to the wall of the target vessel, rotating at constant speed. An OCT imaging method is further mentioned, starting with the catheter insertion into the patient. Subsequently, rotation of the second portion of the catheter with respect to the conduit takes place, which redirects the energy (light) towards the sample and receives the reflected energy for the second portion, redirecting towards the conduit.

The method presented in documents US 8,996,099 B2 and US 2015/0265152 A1 particularly emphasizes the rotative system for directing light towards the tissues to be analysed, i.e., to perform the sample scanning. Although it is also based on the use of the OCT technique, the method presented in document US 8,996,099 does not disclose nor does it suggest the use of video imaging for guiding the developed catheter, that is, it does not combine different imaging diagnostic techniques. It is solely based on the use of OCT to which a rotation system has been coupled.

A method for tissue imaging through the OCT technique is presented in document US8,115,934 B2. This is directed to the evaluation and diagnosis of the ear canal, through the insertion of an optical fibre-based device into the patient's ear. The method includes converting at least one analysis image. Although said method is based on the same optical imaging technique (OCT), it has a different applicability, being directed to the auditory system. Additionally, the exclusive use of the OCT technique for guiding and acquiring the image may hamper the location of abnormalities.

Document US 10,219,780 B2 presents a catheter incorporating two imaging techniques, OCT and intravascular ultrasound, for imaging vasculature systems, including cardiac, peripheral and neuronal vasculature. The OCT system and ultrasound transducer are positioned and assembled at the distal end of a catheter. This system does not concurrently use the conventional colonoscopy and OCT imaging techniques. Furthermore, its field of application relates to cardiac, peripheral and neuronal vasculature.

Documents US 2013/0296695A1 and CA 02767017 present a system for human lumen imaging, which includes a controller and a display. The controller is configured to connect to the proximal end of a fibre optic catheter, allowing the rotation of the catheter end and the collection of the OCT signal through the fibre. The display is configured to show OCT images, corrected through the rotation delay of the coupled catheter. Although said system allows to perform OCT to tissues, it presents as its main drawback the exclusive use of the OCT technique, not being coupled other imaging techniques that help and improve the diagnosis. Through the OCT technique, only an in-depth analysis of the tissues is performed, not being analysed the surface thereof.

Previous studies, M. J. Maciel et al., "Design of a novel TiO2/airgap-based polarizing micro beam splitter cube," J. Phys. Conf. Ser., vol. 1319, no. 1, p. 012006, Sep. 2019 and M. J. Maciel et al., "TiO2/airgap-based polarizing beam splitter: design, simulation, and fabrication based on MEMS technologies," Opt. Lasers Eng., vol. 133, no. 106110, Oct. 2020 disclose the design, simulation and manufacture of a cube beamsplitter for separating the two polarisation states of light. These studies mainly present the design and simulation applied to different wavelengths, being studied and improved the manufacture for the visible region of the electromagnetic spectrum. Furthermore, these studies present a miniaturized cube beamsplitter with an active area of approximately 1.0 x 1.0 mm².

These facts are described in order to illustrate the technical problem addressed by the present invention.

### GENERAL DESCRIPTION

The present invention refers to an analysis system of colorectal tissues which allows the screening, diagnosis, monitoring and a minimally invasive intervention in colorectal tissues through the integration of two image screening techniques in a single device (system). More specifically, the imaging techniques used are: colonoscopy (white light and narrow band imaging) and optical coherence tomography (OCT). In an embodiment, the OCT technology is coupled to a conventional colonoscope, through the insertion of a catheter into the instrument channel thereof. The system allows different levels of diagnosis: (i) initially the white light technique (video) is used for visualizing colorectal tissues through the use of the colonoscope; (ii) suspicious tissues in colonoscopy (i.e., neoplasia identification) are analysed by narrow band imaging (NBI); (iii) those tissues are classified in real-time through an optical biopsy provided by OCT; and (iv) OCT image improvement by using a light polarisation dependent analysis, through the use of a polarizing beamsplitter which separates the -s and -p polarisations of the electromagnetic radiation. In an embodiment, the OCT analysis system uses an assembly in the spectral domain with a superluminescent diode as an optical source and a spectrometer for signal detection. In an embodiment, said system comprises a coupling in the colonoscope instrument channel by means of using optical fibre. In an embodiment, the OCT catheter comprises an optical assembly with lenses, light beamsplitter and reflecting mirrors. In an embodiment, the sample arm reflecting mirror is connected to a micromotor which allows the movement of said reflecting mirror. In an embodiment, the OCT scanning system is comprised of two rotation axes: a first axis which coincides with the mechanical movement of the colonoscope through the patient's rectum and colon, and a second axis which corresponds to the movement of the mirror electronically controlled by the micromotor, allowing a perpendicular movement relatively to the colonoscope direction. Said system allows to identify abnormalities in tissues such as polyps, cysts or other abnormal characteristics of the observed tissue. After the diagnosis provided by the OCT optical biopsy, tissues that make up the large intestine (rectum, colon and cecum) classified as malignant or premalignant can be removed using available instrumentation in conventional colonoscopy, as well as using air and water in the appropriate channels.

An aspect of the present disclosure refers to a system for screening, diagnosis, or monitoring a colorectal tissue for the identification of tissues with morphological alterations, comprising:
a colonoscope (13);
a superluminescent diode (8) as a light source;
a catheter (15) configured to transmit and direct the light from the superluminescent diode (8) towards the colorectal tissue; connected to a spectrometer (9), which comprises a charge-coupled device (10) and a diffraction grating (11) for the detection of a light signal from the colorectal tissues for the optical coherence tomography;
wherein the catheter is inserted into the instrument channel of the colonoscope in order to optically identify neoplasias in the colorectal tissue. The system and method of the present disclosure allows to more accurately identify suspicious malignancy tissues, i.e., with morphological alterations, so as to avoid false negatives, thus reducing the need for further interventions. This system allows to increase the accuracy of neoplasia identification in the colorectal tract tissues, such as for instance polyps, cysts or other abnormal characteristics of the observed tissue.

In an embodiment, the catheter (15) comprises a plurality of micromirrors, a micromotor (16) and a light beamsplitter (18).

In an embodiment, the catheter (15) comprises at its end two micromirrors, preferably with a surface of about 45° (19a and 19b), a third vertical micromirror (19c), a micromotor (16) coupled to the first micromirror (19a), and a light beamsplitter (18).

In an embodiment, the micromotor (16) is configured to move the first micromirror (19a), thus allowing the optical colorectal tissue scanning.

In an embodiment, the catheter (15), i.e., the catheter optical assembly, comprises: a light beamsplitter (18), two lenses coupled (17) to the splitter and a lens (20) fixed to the optical fibre outlet end (21) for guiding the light from the fibre towards the OCT optical assembly.

In an embodiment, the system comprises a polarizing beamsplitter (22), preferably a polarizing micro beamsplitter assembled on a borosilicate glass substrate (26).

In an embodiment, the polarizing beamsplitter (22) comprises a prism and a multilayer (23) deposited on the hypotenuse faces of the prism and which separates two polarisation states of light; preferably wherein the multilayer comprises at least seven layers of thin films.

In an embodiment, the multilayer (23) of the polarizing beamsplitter comprises a low refractive index material and a high refractive index material; the high refractive index material was chosen between silicon dioxide (SiO₂) and titanium dioxide (TiO₂), more preferably TiO₂ because it has a higher refractive index; for the low refractive index material, air was selected because it has lower refractive index among these materials. To create this air layer, a sacrificial layer is used which is then removed leaving air in that space.

In an embodiment, the multilayer (23) of the polarizing beamsplitter comprises 4 titanium dioxide layers (24) and 3 air layers (25).

In an embodiment, the multilayer (23) of the beamsplitter has the following physical thicknesses: 235 nm (TiO₂), 337 nm (air), 161 nm (TiO₂), 506 nm (air), 161 nm (TiO₂), 337 nm (air), 235 nm (TiO₂).

In an embodiment, the optical thickness of the multilayer for the polarizing micro beamsplitter (22) is 1.6H 1.0L 1.1H 1.5L 1.1H 1.0L 1.6H.

In an embodiment, the optical coherence tomography presents an axial resolution of about 12 µm/9 µm (air/water) and a depth of analysis of 7 mm/5.3 mm (air/water).

In an embodiment, the system of the present disclosure is used for the diagnosis, screening, monitoring, or treatment of colorectal tract neoplasias; namely tumours/cancer.

Another aspect of the present disclosure refers to a method for screening, diagnosis, or monitoring a colorectal tissue sample for the identification of tissues with morphological alterations, characterized in that it comprises the following steps:
visualizing the colorectal tract with the colonoscope via the white light technique for identifying suspicious regions;
analysing by means of narrow band imaging and optical coherence tomography in order to identify the morphological and/or in-depth alterations, through OCT cross sectional images.

In an embodiment, the method further comprises a step of obtaining OCT images using a light polarisation dependent analysis.

An aspect of the present disclosure concerns to an analysis system of large intestine tissues which integrates optical coherence tomography (OCT) in conventional colonoscopy for the screening, diagnosis and monitoring, and a minimally invasive intervention in tissues through the insertion of a catheter (15) of about 3.2 to 3.8 mm diameter with an opto-electro-mechanical module, comprising:
an inlet for the instrument channel (1) of a colonoscope (13);
a terminal (2) of the colonoscope (13), which will be guided through the colorectal tract and provided with a first opening (6) for entrance and exit of water and air and a second opening (7) for the instrument channel outlet (1);
water and air ports/controllers (3);
light guides (4) and lenses (5);
a superluminescent diode (8);
a spectrometer (9), provided with a charge-coupled device (10) for signal detection and a diffraction grating (11);
an OCT catheter (15) placed in the second opening (7) of the colonoscope (13) and provided at its end with at least two micromirrors with a surface of about 45° (19a and 19b) and a vertical micromirror (19c);
a micromotor (16) electronically controlled; and
a light beamsplitter (18).

In an embodiment, the micromotor (16) control allows the movement of the first micromirror (19a) for performing the sample scanning (14).

In an embodiment, coupled to the light beamsplitter (18), there are at least two lenses (17) arranged, an additional coupling lens (20) being fixedly positioned at the optical fibre outlet end (21) and used for guiding the light from the fibre towards the OCT optical assembly.

In an embodiment, a polarisation dependent light beamsplitter (22) may be used, assembled on a borosilicate glass substrate (26) and comprising a multilayer (23) formed by at least 7 (seven) layers of thin films deposited on the hypotenuse faces of the prism, which separates the two polarisation states of light.

In an embodiment, the multilayer (23) material is initially selected considering the lowest refractive index material - air and the high refractive index material is selected between silicon dioxide (SiO₂) and titanium dioxide (TiO₂).

In an embodiment, the multilayer (23) is comprised of 4 titanium dioxide layers (24) and 3 air layers (25) and has the following physical thicknesses: 235 nm (TiO₂), 337 nm (air), 161 nm (TiO₂), 506 nm (air), 161 nm (TiO₂), 337 nm (air), 235 nm (TiO₂), ensuring a bandwidth of around 420 nm.

In an embodiment, the multilayer optical thickness for the polarisation dependent light beam microsplitter (22) is: 1.6H 1.0L 1.1H 1.5L 1.1H 1.0L 1.6H.

In an embodiment, the system may allow different diagnosis levels:
i) initially the white light technique (video) is used for visualizing the colorectal tract tissues through the use of the colonoscope for identifying suspicious regions;
ii) identified suspicious tissues are analysed via the narrow band imaging (NBI), performed at the software level of the colonoscope;
iii) tissues are classificated in real-time through an optical biopsy via cross sectional analyses provided by OCT; and
iv) OCT image improvement using a light polarisation dependent analysis, through the use of a polarizing beamsplitter (22) which separates the -s and -p polarisations of the electromagnetic radiation.

In an embodiment, the analysis may present an axial resolution of about 12 µm/9 µm (air/water) and a depth of analysis of 7 mm/5.3 mm (air/water).

In an embodiment, the system allows that after diagnosis, tissues can be removed using conventional colonoscopy instrumentation, as well as using air and water in the appropriate channels.

In an embodiment, initially, images from tissues surface are obtained through the colonoscope video system (white light). In these regions of tissue surfaces it is possible to apply the NBI technique to improve the visualization of certain tissue regions/components. Through these two imaging technologies, the regions of interest are selected for imaging tomography via the optical coherence tomography (OCT) technique. These are the suspicious regions, which would be removed for conventional histopathological analysis (biopsy). Thus, instead of removing them, these regions are additionally analysed in depth, axial section images, through OCT.

The great advantage is the performance of an optical biopsy without the need of removing the tissues, being evaluated the progression of possible tumours in depth and not just on the surface. This analysis is done directly, when performing a colonoscopy. Additionally, using the polarizing beamsplitter, it is surprisingly still possible to increase the contrast of OCT images, useful for identifying and defining tumoural regions, the polarizing beamsplitter allows better identification of altered tissues and minor tissue alterations, namely the identification of microtumours.

### BRIEF DESCRIPTION OF THE DRAWINGS

Features, advantages, technical and functional improvements of the present invention will be better understood by a person skilled in the art from the schematic figures accompanying the present application, which should be construed together with the detailed description below, provided by way of example only of the preferred embodiments of the invention, without any intention of limiting the scope of protection.
**Figure 1** represents a conventional colonoscope, where it is possible to view the inlet to the instrument channel (1), the colonoscope terminal (2), and also the water and air ports/controllers (3).
**Figure 2** is a front view of the colonoscope terminal, containing light guides (4) and lenses (5) of the colonoscope. (6) represents the opening for entrance and exit of water and air. The instrument channel outlet (7) will be the catheter outlet site for OCT analysis.
**Figure 3** represents the concept of the present invention, that is, the integration of the OCT technique into a conventional colonoscope (13).
**Figure 4** represents the OCT analysis and diagnosis catheter end, with the opto-electro-mechanical assembly.
**Figure 5** represents the optical assembly with the introduction of the polarizing cube beamsplitter (22), which separates the two polarisation states of light.
**Figure 6** shows the scheme of the polarizing beamsplitter.

### DETAILED DESCRIPTION

The present invention refers to an analysis system of large intestine tissues, especially colorectal tissues, which allows the screening, diagnosis and monitoring, and a minimally invasive intervention in tissues through the integration of two image screening techniques in a single device. More specifically, the imaging techniques used are: colonoscopy (white light and narrow band imaging) and optical coherence tomography (OCT).

The OCT technology is coupled to a conventional colonoscope through the insertion of a catheter into the instrument channel thereof. In this catheter an opto-electro-mechanical module is miniaturized for directing light towards the sample of interest and it has dimensions compatible with the instrument channel diameter, which can range from about 3.2 to 3.8 mm. Thus, for a preferred embodiment of the present invention, the catheter used shows a diameter of approximately 3.2 mm, being compatible with most colonoscopes.

In an embodiment, the system allows different levels of diagnosis: (i) initially the white light technique (video) is used for visualizing colorectal tract tissues through the use of the colonoscope for the identification of suspicious regions; (ii) the suspicious tissues identified during colonoscopy are analysed via the narrow band imaging (NBI), performed at the software level of the colonoscope; (iii) those tissues are classified in real-time through an optical biopsy provided by OCT; and (iv) improvement of OCT images using a light polarisation dependent analysis, through the use of a polarizing beamsplitter which separates the -s and -p polarisations of the electromagnetic radiation.

In an embodiment, the analyses performed in step (iii) are cross sectional analyses, commom to those skilled in the art of computed axial tomography (CAT) or magnetic resonance. Specifically, in a preferred embodiment of the present invention, the cross section performed has an axial resolution of about 12 µm/9 µm (air/water) and a depth of analysis of 7 mm/5.3 mm (air/water). Those values are compatible with the colorectal tissue. However, the invention is not limited to this type of visualization, and other cross sections can be obtained by imaging techniques, at the software level. The resolution values for the OCT system used are suitable for colonoscopy applications, offering enough resolution for the identification abnormalities in tissues. The analysis system uses an assembly in the spectral domain through the use of an optical source centred at a wavelength of about 1325 nm and a spectrometer for signal detection positioned on the detection arm. The coupling in the colonoscope instrument channel is implemented through the use of optical fibre as a light guide between the optical source and the catheter end, where a micro-opto-electro-mechanical module for guiding the light towards the tissues is assembled. In an embodiment, the optical source, spectrometer and detection system are kept externally to the colonoscope. Said module comprises an optical assembly with lenses, light beamsplitter and reflecting micromirrors for total reflection of light. The sample arm reflecting mirror is connected to a micromotor that allows the movement of said reflecting mirror. Thus, the OCT scanning system is composed of two rotation axes: a first axis which coincides with the mechanical movement of the colonoscope, with the OCT analysis catheter located at the outlet end of the colonoscope instrument channel. Said movement occurs through the patient's rectum and colon. On the other hand, the second rotation axis corresponds to the mirror movement electronically controlled by the micromotor, allowing a movement perpendicular to the colonoscope direction. The system thus allows a three-dimensional scanning, that is, the two axes plus the in-depth analysis achieved by OCT.

Additionally, the system may comprise a polarizing cube beamsplitter which separates the two polarisation states of light, providing a more complete OCT analysis by highlighting certain areas, i.e., increasing the contrast in images, correlated with the polarisation state of the light reflected from the sample.

In an embodiment, the polarizing cube beamsplitter is assembled on a glass substrate, the multilayer being formed by at least 7 (seven) layers of thin films on the hypotenuse faces of the prism. The layers are arranged interleavedly so that a first layer formed by a high refractive index material is alternated with a layer formed by a low refractive index material. A person skilled in the art understands that the refractive index varies according to the wavelength value. For a preferred embodiment of the present invention, the wavelength chosen corresponds to the OCT source wavelength, that is, 1325 nm. Specifically, the material of said layers was initially selected considering the lowest refractive index material. In this regard, air has been selected as the low index material (L). On the other hand, the high refractive index material is selected between silicon dioxide (SiO₂) and titanium dioxide (TiO₂). For a preferred embodiment of the present invention, the selected material was TiO₂ for presenting a higher refractive index relative to SiO₂. However, a person skilled in the art understands that the option of using TiO₂ does not represent a limitation of the present invention and that SiO₂, or any other material that performs the function effectively, could be an option of use, considering, however, a different number of layers that would almost certainly be superior if SiO₂ was used. Additionally, TiO₂ shows a high transmittance in the visible zone, high dielectric constant and high chemical stability.

In an embodiment, for a preferred implementation of the present invention, the optical thickness of the multilayer (expressed in multiples of one-fourth of the central wavelength) for the polarizing micro beamsplitter is the following: 1.6H 1.0L 1.1H 1.5L 1.1H 1.0L1.6H.

In an embodiment, the polarizing cube beamsplitter is manufactured using common manufacturing techniques for standard micro-electro-mechanical systems (MEMS), such as: deposition of thin films of the material of interest (titanium dioxide) and of sacrifice layers, removed subsequently by a corrosion method for obtaining the air layers. The sacrifice material is selected among different options, like chromium, aluminium, silver or gold. For the present embodiment the material used was chromium, because it shows adhesion to titanium dioxide. The manufacturing process also includes blade cutting of the glass plates (wafers), so as to obtain the cubic configuration.

In an embodiment, one of the seven layers is a resonance layer (thickness oriented to the wavelength to be transmitted), the 3 layers on each side have symmetry in terms of their thickness value, the manufacturing process of the filter being performed by radiofrequency sputtering. In a preferred embodiment of the present invention, the multilayer of the polarizing beamsplitter has the following physical thicknesses: 235 nm (TiO₂), 337 nm (air), 161 nm (TiO₂), 506 nm (air), 161 nm (TiO₂), 337 nm (air), 235 nm (TiO₂). These thicknesses ensure a bandwidth of around 420 nm. These thickness values are obtained with the refractive indexes of a TiO₂ thin film, manufactured by radiofrequency sputtering. A person skilled in the art understands that these thicknesses may vary according to the refractive index values, since the later are affected by the deposition process used.

It is worth mentioning that the entire process of thin films deposition is performed using shadow masks, manufactured for the purpose of manufacturing the present beamsplitter. In addition to the deposition process steps, an annealing process of the thin films deposited is further performed. That step performed provides a better structure and reduces the residual stress of the titanium dioxide films.

In an embodiment, after the diagnosis provided by the OCT optical biopsy, tissues classified as malignant or premalignant can be removed using instrumentation available in conventional colonoscopy, as well as using air and water in the appropriate channels.

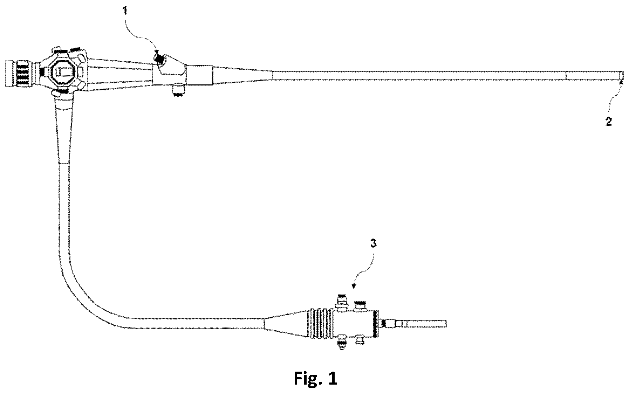

In an embodiment, a conventional colonoscope model is shown in Figure 1. In that model, it is possible to view the inlet for the instrument channel (1), the terminal (2) of the colonoscope (13), which will be led through the colorectal tract, and also the water and air ports/controllers (3) which will assist during the colonoscopy procedure.

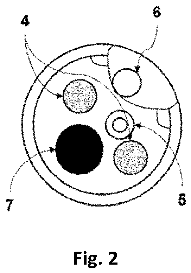

In an embodiment, the terminal (2) of colonoscope (13) is comprised of light guides (4) and lenses (5) of the colonoscope for imaging and minimally invasive instrument guidance. Said terminal also has a first opening (6) for entrance and exit of water and air and a second opening (7) for the instrument channel outlet (1), which coincides with the catheter outlet site for OCT analysis, as shown in Figure 2.

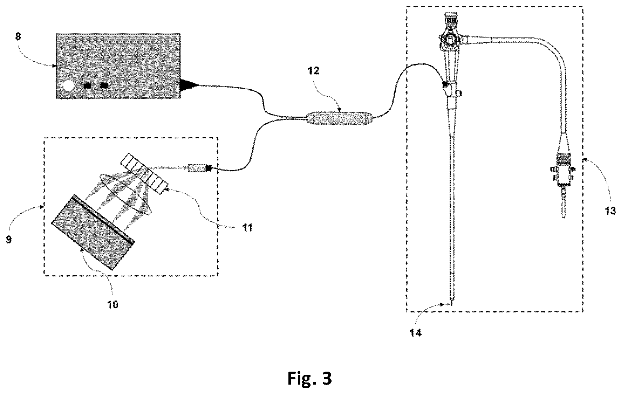

In an embodiment, the entire system of the present invention is shown in detail in Figure 3. Said figure shows the integration of the OCT technique in a conventional colonoscope (13). Specifically, the OCT assembly is a spectral domain approach, using a superluminescent diode (8) as an optical source and a spectrometer (9) as an OCT detection system.

In an embodiment, in the spectrometer (9) there is a charge-coupled device (10) for signal detection and a diffraction grating (11). The connection between the spectrometer (9) and the colonoscope (13) is made by means of an optical fibre connector (12), which serves as an intermediary among the superluminescent diode (8), the spectrometer (9) and the colonoscope (13). The OCT catheter (15) located at the outlet end of the second opening (7) of the instrument channel (1) at the terminal (2) of the colonoscope (13) allows the emission and direction of light towards the sample (14) of interest to be analysed.

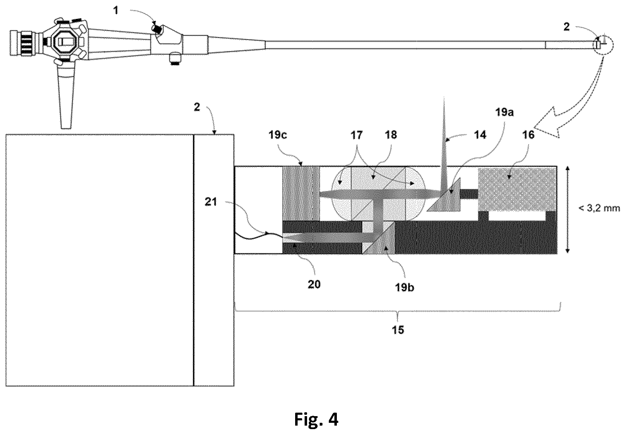

In an embodiment, the catheter end (15) of the OCT analysis and diagnosis with the opto-electro-mechanical assembly is shown in detail in Figure 4. Specifically, that end of the catheter is composed of at least two micromirrors with a surface of about 45° (19a and 19b) and a vertical micromirror (19c), which allows the total reflection of light. The micromotor (16) electronically controlled allows the movement of a first micromirror (19a) for performing the scan on the sample.

In an embodiment, the light beamsplitter (18) allows the light separation towards the reference arm (light guided towards mirror 19c) and towards the sample to be analysed (light guided towards mirror 19a and subsequently out of the catheter (15)). Coupled to the light beamsplitter (18), there are arranged at least two lenses (17). An additional coupling lens (20) is fixedly positioned at the optical fibre outlet end (21) and is used for guiding the light from the fibre towards the OCT optical assembly.

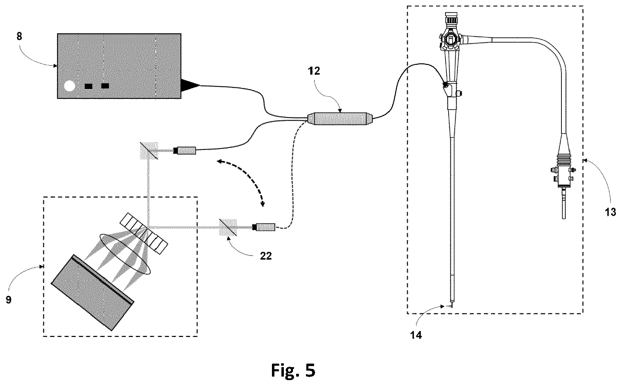

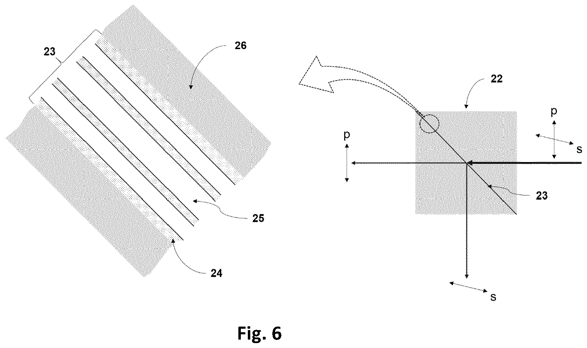

In an embodiment, the final optical assembly further comprises the introduction of a polarizing cube beamsplitter (22), represented in Figure 5 and more in detail in Figure 6, which separates the two polarisation states of light (-s and -p polarisations). This element, which is preferably cubic, but can assume any other form as long as it allows the perpendicular separation of the two polarisation states of light, provides an increased OCT diagnostic capability. In a preferred embodiment of the present invention, the polarizing cube beamsplitter (22) is manufactured on a borosilicate glass substrate (26) with a multilayer (23) deposited on the hypotenuse faces of the prism which separates the two polarisation states of light. However, it is evident that the substrate material may be selected from any other type of transparent material which allows light to pass through, after separation of its polarisation states on the deposited multilayer.

In an embodiment, in a preferred implementation of the present invention, the multilayer (23) is composed of 4 titanium dioxide layers (24) as a high refractive index material, and 3 air layers (25) as a low refractive index material.

The present invention is, of course, in no way restricted to the embodiments described in this document and a person with ordinary skill in the art may foresee many possibilities of modification thereof and of replacements of technical features for other equivalent, depending on the requirements of each situation, as defined in the appended claims.

The following claims define additional embodiments of the present description.

## Claims

1. System for screening, diagnosis, or monitoring a colorectal tissue for the identification of tissues with morphological alterations comprising:
a colonoscope (13);
a superluminescent diode (8) as a light source;
a catheter (15) configured for transmiting and directing the light from the superluminescent diode (8) towards the colorectal tissue; connected to a spectrometer (9) comprising a charge-coupled device (10) and a diffraction grating (11) for the detection of a light signal from the colorectal tissues for the
optical coherence tomography;
wherein the catheter is inserted into the instrument channel of the colonoscope in order to optically identify neoplasias in the colorectal tissue.

2. System according to the previous claim, wherein the catheter (15) comprises a plurality of micromirrors, a micromotor (16) and a light beamsplitter (18).

3. System according to any one of the previous claims, comprising a polarizing beamsplitter (22), preferably a polarizing micro beamsplitter assembled on a borosilicate glass substrate (26).

4. System according to the previous claim, wherein the polarizing beamsplitter comprises a prism and a multilayer deposited on the hypotenuse faces of the prism which separates the two polarisation states of light (23); preferably wherein the multilayer comprises at least seven layers of thin films.

5. System according to the previous claim, wherein the multilayer (23) of the polarizing beamsplitter is comprised of a low refractive index material and a high refractive index material; the high refractive index material was chosen between silicon dioxide and titanium dioxide, more preferably titanium dioxide, and the low refractive index material chosen was air.

6. System according to any one of claims 4 and 5, wherein the multilayer (23) of the polarizing cube beamsplitter comprises 4 titanium dioxide layers (24) and 3 air layers (25).

7. System according to any one of claims 4-6, wherein the multilayer (23) of the polarizing beamsplitter has the following physical thicknesses: 235 nm (TiO₂), 337 nm (air), 161 nm (TiO₂), 506 nm (air), 161 nm (TiO₂), 337 nm (air), 235 nm (TiO₂).

8. System according to any one of claims 4-7, wherein the optical thickness of the multilayer for the polarizing micro beamsplitter (22) is 1.6H 1.0L 1.1H 1.5L 1.1H 1.0L 1.6H.

9. System according to any one of the previous claims, wherein the optical coherence tomography presents an axial resolution of about 12 µm/9 µm (air/water) and a depth of analysis of 7 mm/5.3 mm (air/water).

10. System according to any one of the previous claims, wherein the catheter (15) comprises at its end two micromirrors, preferably with a surface of about 45° (19a and 19b), a third vertical micromirror (19c), a micromotor (16) coupled to the first micromirror (19a).

11. System according to any one of the previous claims, wherein the micromotor (16) is configured for moving the first micromirror (19a), thus allowing the optical scanning on colorectal tissues.

12. System according to any one of the previous claims, wherein the catheter (15) comprises: a light beamsplitter (18), two coupled lenses (17) and a lens (20) fixed to the optical fibre outlet end (21) for guiding the light from the fibre towards the OCT optical assembly.

13. System according to any one of the previous claims for the diagnosis, screening, monitoring, or treatment of neoplasias of the colorectal tract; namely tumours.

14. Method for screening, diagnosis, or monitoring a colorectal tissue sample for the identification of tissues with morphological alterations according to any one of claims 1-13, **characterized in that** it comprises the following steps:
viewing the colorectal tract with the colonoscope via the white light technique to identify suspicious regions;
analysing by means of narrow band imaging and optical coherence tomography in order to identify the morphological and/or in-depth alterations.

15. Operating method according to the previous claim, further comprising a step of obtaining OCT images using a light polarisation dependent analysis.
